# EUROPEAN PATENT APPLICATION

(11) **EP 2 255 741 A1**
(43) Date of publication of application: **01.12.2010**
(21) Application number: 09175451.5
(22) Date of filing: 09.11.2009
(51) Int. Cl.: A61B 18/02

(54) **Cryoprobe for cryosurgical procedures**

(30) Priority: 27.05.2009 PL 38812909
(71) Applicant: Nitek, Stanislaw, 05-091 Zabki (PL)
(72) Inventor: Nitek, Stanislaw, 05-091 Zabki (PL)
(74) Representative: Kaminski, Piotr

(57) **Abstract**

Object of the invention is a cryoprobe for cryosurgery, **characterised in that** it is equipped with a movable longitudinal element (1), the element being located in one plane with the cryoprobe cryohead (2) and forms with it a system of forceps.

## Description

The invention relates to a cryoprobe for cryosurgery, particularly for laryngological surgery.

Cryosurgery and cryoprobes used in that area (applicators of freezing means, freezing tips) are known and usually have a shape similar to a ball-point pen or are slightly curved. Body of the cryoprobe is slightly wider than a cryohead and during operation it is fastened to the tip feeding with cooling agents, which can be e.g. dinitrogen monooxide. The feeding tip is integrated with a handle (handgrip) of the cryoprobe made of plastic, which usually includes elements controlling cryoprobe operation. Depending on its purpose, the cryoprobe body can be slightly bent or have an "S"-shape. Usually, the cryohead is slightly narrower than the body and has a gently rounded tip. It is possible to modify the cryoprobe head depending on its use; they can have different thickness and more rounded tip or thinner and conical or wider shape (like a flattened drop).

Examples of cryosurgical devices were disclosed e.g. in utility models PL57773, PL56225 and PL62173. The devices are usually equipped with tips having a straight body and have rounded cryoheads. A cryoapplicator presented in the description of utility model PL57966, has a handle, an arm and a freezing part and is **characterised in that** the axle of the freezing part is parallel to the axle of the handle, the axles being distant from each other by a dimension "A" to allow free grip with hand and an applying a force axially when performing operations, moreover a cryoapplicator arm has at least two flexures (a shape of an extended letter "S").

Typical cryoprobes do not make it possible to keep simultaneously tissue to be operated in place, as the only element utilised at operations are freezing tips. The cryoprobes are not equipped with any additional mechanical elements that facilitate certain types of operations.

Object of the invention is a cryoprobe for cryosurgery, **characterised in that** it is equipped with a movable longitudinal element 1, the element being located in one plane with the cryohead 2 and forming together with it a system of forceps.

By means of a pull rod 5 and/or a lever 4, the longitudinal element 1 is joined with a handle 9 and/or a release mechanism of a pull rod 10.

The handle 9 is located in a plane perpendicular to a plane in which the cryohead 2 and the longitudinal element 1 are positioned.

The pull rod 5 and/or the lever 4 are located along the cryoprobe body 6 and are connected with them by means of at least two clamping rings 7 and 8.

The longitudinal element 1 has a shape of a straight rod or a bent rod and is optionally flattened. It can also be equipped with protrusions of the length of 2 to 5 mm located on the edge of a movable element from the cryohead side. Preferably, both the movable element and the head are equipped with protrusions located approximately vis-a-vis each other, so that they touch each other during operation and make it easier to capture of tissue being operated.

The cryoprobe of the invention, as distinct from other known types, makes it possible to capture precisely, clamp and freeze tissues when a use of a typical model of a cryoprobe may not work. Addition of a mechanical system that controls operation of the longitudinal element 1 allows to clamp it to the cryohead in a controllable way. The cryoprobe of that type may, for example, be suitable in laryngological operations, e.g. while cutting frenulum of the tongue in children. Although surgery is nearly painless, usually it is not possible to eliminate anxiety felt by children. Violent movements may cause that the surgery is not accurate and unnecessarily takes more time. The precise, holding movement of the system of forceps, achieved by modification of the cryoprobe makes it possible to overcome these problems. It also allows to eliminate additional instruments during surgery, manipulation with which is difficult during cryoprobe operation. The modified cryoprobe is evidently suitable also in cryosurgical operations that require holding a tissue section being operated. These can be exophytic changes of any type, stalked like nasal polyps or haemorrhoidal varices.

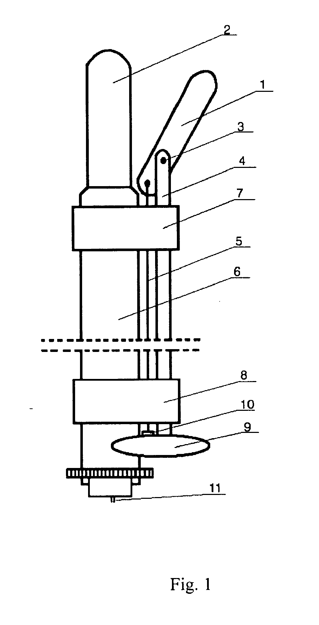

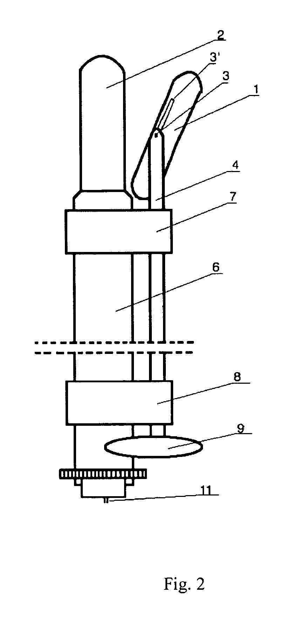

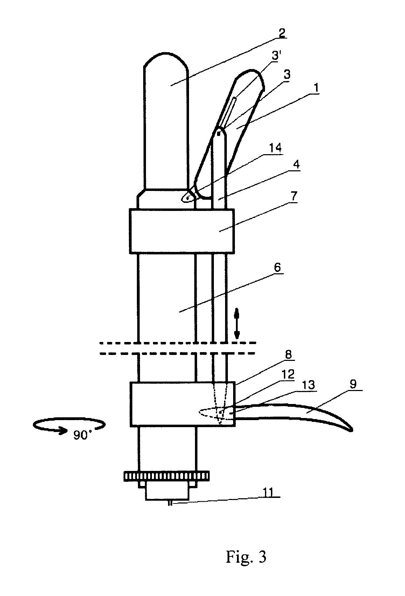

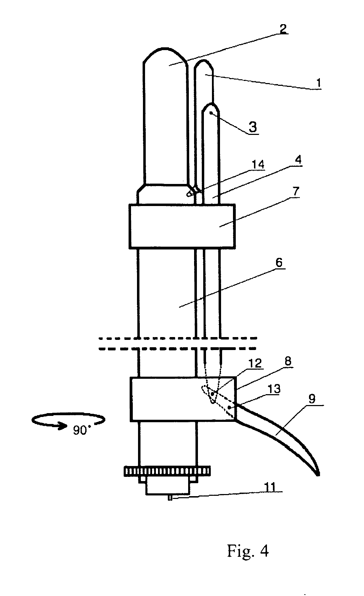

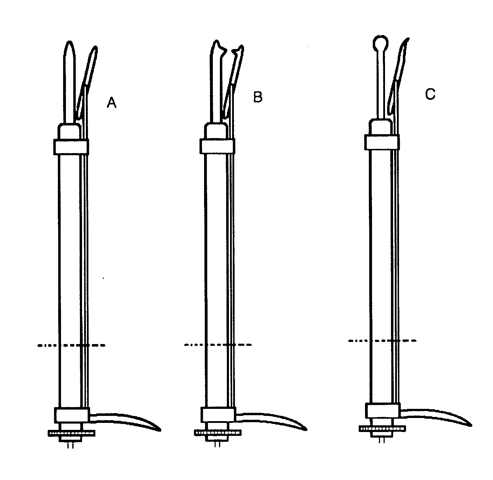

Object of the invention is presented in an embodiment on a drawing, where Fig. 1 presents a schematic side view of the cryoprobe equipped with the pull rod and the lever, Fig. 2 - cryoprobe equipped with the lever, Fig. 3 presents the cryoprobe with an exemplary mechanism of transmission of handle movement to the lever, its part including the handle being rotated by 90° in relation to the cryohead, Fig. 4 shows the cryoprobe of Fig. 3, its movable element being pressed against the cryohead, Fig. 5 presents a variation of the cryoprobe having other shapes of the cryohead and the movable element 1. On Figs. 3, 4 and 5, a part including the handle being rotated by 90 degrees in relation to a part including the head is shown, so as to visualise the cryoprobe handle. A vertical arrow on Fig. 3 shows a movement of the lever pushing the element 1. A horizontal broken line denotes schematic "cut" of the cryoprobe body, to make visible the structure of its upper and lower part. In general, the structure of the body itself and its length may be arbitrary.

The cryoprobe of the invention is made of the body 6, on the lower ending of which there is a feeder cable 11, and on an upper ending there is the cryohead 2. Next to the head 2 there is a movable longitudinal element 1 connected with the lever 4 with the joint 3. The lever 4 is situated along the body 6 and it is connected with it by means of the clamping rings 7 and 8. At the lower part of the cryoprobe body an ergonomical handle 9 is located.

In another embodiment the cryoprobe is equipped with the lever 4 and the pull rod 5. As distinct from the first variant of the cryoprobe, both elements together are responsible for operation of the movable element 1. In this variant, the cryoprobe is equipped with the handle 9 and the release mechanism 10.

In the cryoprobe, in addition to typical elements, a movable system of the longitudinal element 1 has been applied, actuated by the lever 4, which transmits pressing exerted by fingers onto the handle 9, while pressing the element 1 against the head 2 of the cryoprobe. Thanks to it, an effect is achieved such as in the system of forceps, i.e. effect of clamping the tissue between two edges - the inner edge of the element 1 and the inner edge of the head 2. Thus, the definition according to which the element 1 and the head 2 form a system of forceps means that the element 1 performs such a movement as in the system of forceps, of a known medical instrument, in which its arms are clamped in a forceps-type movement. The cryohead is mounted as fixed in the cryoprobe body. In clamping rings 7 and 8 there are openings that make possible the free longitudinal movement of the lever 4 which is pushed by the handle 9. Then, the joint 3 is shifted along the canal 3'. Such type of the cryoprobe is shown on Fig. 2. In another embodiment of the invention, the lever is located so that pulling the handle 9 does not produce a push of the lever, but also results in its pulling. Such variant requires only slight changes in a way the element 1 is connected with the lever. In addition the cryoprobe may be optionally, equipped with the pull rod 5 ended with the release mechanism 10 located near the handle 9 to enable convenient and precise operation of the device simultaneously by means of the handle 9 and the release mechanism 10.

In Figs. 3 and 4, the advantageous example of the cryoprobe is shown, in which the lever 4 is connected with the handle 9 through the movable joint 12. Thanks to that the cryoprobe is equipped with the joint 13, movement of the handle 9 is in a simple way transmitted into the element 1. In Fig. 3 and 4, a connection between the lever and the handle inside the clamping ring 8 is shown with broken line. The handle can be additionally stabilised with a joint 13, whereas the movable element 1 - with joint 14. The above-mentioned joints can have different structure. In general, these are mandrels mounted in openings, so that they form a movable (hinged) joint. Two joined elements can freely move during operation. The joint 13 enables movable mounting and fixing of the handle 9 to the clamping ring 8, and the joint 14 fixes the element 1 to the body 6.

In Fig. 5, exemplary different shapes of the cryoprobe head and the movable element 1 are shown (Fig. 5: A, B and C). They can be slightly curved (C) and can have different endings e.g. rounded endings, as in case of the cryoprobe head C. A variant of the cryoprobe equipped with protrusions located on the edges of the cryohead and the movable element touching each other is also advantageous (Fig. 5, Example B). These protrusions can have length within the range from 1 to 3mm, e.g. 1 or 2mm. In another embodiment, edges of the cryoprobe head and/or of the element 1 can be equipped with two protrusions or with several smaller protrusions arranged one below another.

The essential aspect of the cryoprobe of the invention is its modification that consists in adding the movable element 1 holding tissue during surgery. Mechanical elements enabling the "forceps-type" movement of the element 1 are indispensable, but their structure is of secondary significance and can be arbitrary. One skilled in the art will know how to adapt known solutions employed in medical equipment of other type. Moreover, the handle used in the lower part of the cryoprobe should be convenient in manipulating and have ergonomical shape. Preferably, because of the handle 9 the cryoprobe looks like a pistol. Fingers enclose the handle optionally together with the release mechanism. The handle is located in a plane perpendicular to a plane in which movement of the element 1 pressed against the head edge 2 takes place. Thus e.g. during surgery of cutting frenulum of the tongue in children, the cryoprobe handle held in hand is directed downwards, the cryoprobe body is located in the oral cavity, and the movable longitudinal element 1 moves under the tongue at a surface parallel to it. Preferably, the cryoprobe employed in such operations should be slightly shorter than a typical one and have length e.g. 5-15 cm e.g. 8, 10 or 12cm. Such construction of the cryoprobe makes it possible to operate conveniently the device during surgery. Movable element mechanically pressed against the cryoprobe head can have length of several milimeters or 1 - 1,5cm and essentially is within the range from 5 to 30 mm. It is merely preferable variant of the cryoprobe of the invention and does not limit its use to other types of surgery. Then, the handle can have slightly different construction. The cryohead of the cryoprobe is covered with noble metals, such as platinum, gold. The movable element , which is essentially not directly fed with freezing substances can yet be frozen e.g. through contact with the head, thus preferably it should also be covered with noble metal.

## Claims

1. Cryoprobe for cryosurgery, **characterised in that** it is equipped with a movable longitudinal element (1), the element being located in one plane with cryohead (2) of the cryoprobe and forming with it a system of forceps.

2. Cryoprobe according to claim 1 **characterised in that** the longitudinal element (1) is joined by means of the pull rod (5) and/or the lever (4) with the handle (9) and/or the release mechanism of the pull rod (10).

3. Cryoprobe according to claim 1, **characterised in that** the handle (9) is located in a plane perpendicular to a plane in which the cryohead (2) and the longitudinal element (1) are located.

4. Cryoprobe according to claim 1 **characterised in that** the pull rod (5) and/or the lever (4) are located along the cryoprobe body (6) and are connected with it with at least two clamping rings (7) i (8).

5. Cryoprobe according to claim 1 **characterised in that** the longitudinal element (1) has a shape of a simple rod or a bent rod and is optionally flattene
